(19) 

Europäisches Patentamt
European Patent Office
Office européen des brevets

(11) **EP 4 265 611 A2**

(12) **EUROPEAN PATENT APPLICATION**
published in accordance with Art. 153(4) EPC

(43) Date of publication:
**25.10.2023  Bulletin 2023/43**

(21) Application number: **22902459.1**

(22) Date of filing: **07.07.2022**

(51) International Patent Classification (IPC):
*C07D 403/14* (2006.01)     *C09K 11/06* (2006.01)
*A61K 49/00* (2006.01)     *A61P 35/00* (2006.01)

(52) Cooperative Patent Classification (CPC):
**A61K 49/00; A61P 35/00; C07D 403/14;**
**C09K 11/06**

(86) International application number:
**PCT/CN2022/104400**

(87) International publication number:
**WO 2023/138005 (27.07.2023 Gazette 2023/30)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **19.01.2022  CN 202210060838**

(71) Applicant: **Guangzhou Shiwen Biotechnology Co.,**
**Ltd.**
**Guangzhou, Guangdong 510425 (CN)**

(72) Inventor: **GAO, Xin**
**Guangzhou**
**Guangdong 510630 (CN)**

(74) Representative: **Cabinet Chaillot**
**16/20, avenue de l'Agent Sarre**
**B.P. 74**
**92703 Colombes Cedex (FR)**

(54)     **SYNTHESIS METHOD AND APPLICATION OF PSCA-SPECIFIC NEAR-INFRARED PROBE**
**GGA-ICG**

(57)     The present invention describes a method for synthesizing a near-infrared probe GGa-ICG that specifically binds to PSCA. The method involves isolating and identifying a nucleic acid aptamer from a prostate cancer cell line that exhibits specific binding to PSCA, and conjugating the aptamer with a near-infrared fluorescent dye to obtain the PSCA-specific binding near-infrared probe GGa-ICG.

## Description

### Technical Field

The present invention relates to the field of biomedical detection technology, and specifically to a method for synthesizing and applying a near-infrared probe GGa-ICG that binds specifically to PSCA.

### Background

Early diagnosis of solid tumors, as well as accurate staging, are crucial for prognosis. In clinical surgery, precise removal of tumors while preserving more healthy tissue is an important factor affecting patients' long-term survival and quality of life after surgery.

Currently, the incidence of tumors in China is on the rise, such as bladder cancer, gastric cancer, and prostate cancer, and the incidence rate continues to increase, posing a serious threat to the health of the Chinese people. Unlike Western countries, the incidence characteristics of these tumors in China are that most patients are already in the advanced or late stage when they seek medical treatment. For advanced cases, treatment requires balancing the complete removal of the tumor and its invasive lesions, including the primary tumor and metastatic lymph nodes, while preserving more healthy tissue and avoiding serious complications, which is a major challenge in treatment, especially in surgery. Therefore, if the occurrence and invasion of tumors, the site and size of metastasis, and the scope and degree of invasion can be accurately assessed, it would have significant implications for clinicians to make rational choices for surgery and treatment plans. For late-stage patients, accurately assessing the extent of invasion and tumor progression is an important basis for selecting and evaluating treatment options. Therefore, there is an urgent need for tumor probes that can specifically bind to tumor tissues and provide intraoperative visualization and postoperative assessment of tumor specificity.

Near-infrared dyes, particularly indocyanine green (ICG), are currently widely used intraoperative fluorescence imaging agents in clinical practice. Many studies have utilized ICG to visualize sentinel lymph node dissection for tumor metastasis. However, the binding of ICG to tumors is due to the enhanced permeability and retention (EPR) effect in tissues, which lacks tissue specificity, resulting in low sensitivity and specificity. To achieve tumor-specific binding, molecules with specificity need to be connected. Currently, commercially available ICG has a relatively stable chemical structure and cannot be directly linked to new targeting molecules, such as antibodies.

PSCA (prostate stem cell antigen) is widely expressed in multiple tumors, commonly found in prostate cancer, renal cell carcinoma, bladder cancer, lung cancer, breast cancer, colon cancer, etc. Currently, there are antibody complexes linked with small molecular antibody fragments targeting PSCA in the development stage. However, due to the intrinsic nature of antibody proteins, immune reactions cannot be avoided, and they are susceptible to proteases such as protein hydrolases in the body. Nucleic acid aptamers are single-stranded oligonucleotide molecules that function similarly to antibodies and antibody-like substances. They can specifically recognize a variety of targets, including metal ions, organic small molecules, peptides, proteins, cells, etc. Their affinity is comparable to or even higher than that of antibodies. Aptamers are small in size, do not carry genetic information, have no immunogenicity, and have stable chemical properties. They are easy to synthesize and have minimal batch differences, which are advantages that cannot be replaced by antibodies. Therefore, nucleic acid aptamers have broad prospects in biotechnology, disease diagnosis, and targeted therapy.

### Summary

The present invention aims to address the limitations of existing technologies and provides a method for synthesizing and using a near-infrared probe GGa-ICG that specifically binds to PSCA. Based on a prostate cancer cell line, a nucleic acid aptamer that specifically binds to PSCA was isolated and identified. It was then linked with a near-infrared fluorescent dye to obtain a near-infrared probe GGa-ICG that specifically binds to PSCA.

To achieve the above objectives, the present invention employs the following technical solution:

A method for synthesizing a PSCA-specific binding near-infrared probe GGa-ICG is provided. The synthesis method includes the following steps:

Step 1:
Into a microwave vessel were added 1,1,2-trimethyl-1H-benzo[e]indole, 7-bromohexanoic acid, potassium iodine and o-dichlorobenzene. The vessel was sealed with a septum and placed into the microwave cavity. The reaction mixture was kept at 145 °C for 90 min. The mixture was worked up and purified after reaction completion to afford the target compound 3-(5-carboxypentyl)-1,1,2-trimethyl-1H-benzo[e]indol-3-ium iodide as gray solids with 47% yield.

S2:

4-(1,1-dimethyl-1H-benzo[e]indol-3-ium-3-yl)butane-1-sulfonate was dissolved in acetic anhydride. Then N-((1E,3E,5E)-5-(phenylimino)penta-1,3-dien-1-yl)aniline was added and the mixture was stirred at 120°C for 5 hours. After cooling to room temperature,3-(5-carboxypentyl)-1, 1,2-trimethyl-1H-benzo[e]indol-3-ium iodide was dissolved in pyridine was added dropwise. After dropping completion, the mixture was stirred at 50°C for 2 hours. The mixture was quenched by hydrochloric acid and subsequently purified to afford the target compound 4-(2-((1E,3E,5E,7E)-7-(3-(5-carboxypentyl)-1,1-dimethyl-1,3-dihydro-2H-benzo[e]in dol-2-ylidene)hepta-1,3,5-trien-1-yl)-1,1-dimethyl-1H-benzo[e]indol-3-ium-3-yl)buta ne-1-sulfonate as purple red solid with 17% yield.

S3:
Compound

4-(2-((1E,3E,5E,7E)-7-(3-(5-carboxypentyl)-1,1-dimethyl-1,3-dihydro-2H-benzo[e]in dol-2-ylidene)hepta-1,3,5-trien-1-yl)-1,1-dimethyl-1H-benzo[e]indol-3-ium-3-yl)buta ne-1-sulfonate, N-(3-dimethylaminopropyl)-N'-ethylcarbodiimide hydrochloride (EDCI) and N-Hydroxysuccinimide(NHS) were dissolved in dichloromethane (DCM). After stirring at room temperature for 5 hours, the reaction was worked up and purified to afford the target compound 2-[(1E,3E,5E)-7-[(2E)-3-{6-[(2,5-dioxypyrrolidine-1-yl)O]-6-(caproyl)}-1,1-dimethyl -1H,2H,3H-benzo[E]indole-2-ylidene]penta-1,3,5-triene-1-yl]-1,1-dimethyl-3-(4-sulfo natobutyl)-1H-benzo[E]indole-3-azole as dark green solid with 42% yield.

S4:
2-[(1E,3E,5E)-7-[(2E)-3-{6-[(2,5-dioxypyrrolidine-1-yl)O]-6-(caproyl)}-1,1-dimethyl -1H,2H,3H-benzo[E]indole-2-ylidene]penta-1,3,5-triene-1-yl]-1,1-dimethyl-3-(4-sulfo natobutyl)-1H-benzo[E]indole-3-azole, $GGa(NH_2C_6)$ were dis-solved in the buffer solution (pH= 9.18).The suspension was stirring at room temperature for 24 hours. After reaction, the mixture was purified by dialysis and lyophilization to afford the target compound GGa-ICG as a dark green solid.

The present invention also provides an application of the obtained near-infrared probe GGa-ICG in detecting PSCA. It can specifically bind to PSCA, be internalized by cells via the clathrin pathway, and a near-infrared fluorescent signal can be detected within 48 hours in the target cells.

It should be noted that the penetration depth of the near-infrared fluorescent probe GGa-ICG can reach 6mm.

It should be noted that the binding site of the near-infrared probe GGa-ICG to PSCA protein is located in amino acids14-28 , i.e., ALQPGTALLCYSCKA.

It should be noted that the near-infrared fluorescent probe GGa-ICG can be used for specific detection of PSCA in prostate cancer, bladder cancer, renal cancer, breast cancer, colon cancer, lung cancer, and pancreatic cancer.

The beneficial effects of the present invention are that:

1. It is able to effectively bind specifically to PSCA.

2. The near-infrared fluorescent probe GGa-ICG can be internalized via the clathrin pathway and can detect near-infrared fluorescence signals within the target cells within 48 hours.

3.Compared to ordinary ICG probes, the near-infrared fluorescent probe GGa-ICG of the present invention has a penetration depth of up to 6mm.

4.The water solubility of the near-infrared fluorescent probe GGa-ICG is improved compared to existing ICG, and it is partially metabolized via the liver and kidney pathways. As a fluorescent contrast agent, it can provide a higher signal-to-noise ratio and a longer observation window time.

5.The PSCA near-infrared fluorescent probe GGa-ICG of the present invention possesses the capability to identify and diagnose various types of tumors.

**Brief Description of Figures**

Figure 1 shows the specific binding of GGa-ICG to prostate cancer cell lines in the present invention.

Figure 2 shows the specific binding of GGa-ICG to prostate cancer cell lines in the present invention. FIGS.2A-2D demonstrate the specific binding of GGa-ICG to PC3 cells, FIG. 2E shows the specific binding of GGa to prostate cancer cell lines, FIG.2F demonstrates the high affinity of GGa to target cells, FIG.2G-2H show that GGa-ICG does not affect cell growth, and FIG.2I demonstrates that GGa-ICG enters cells through endocytosis.

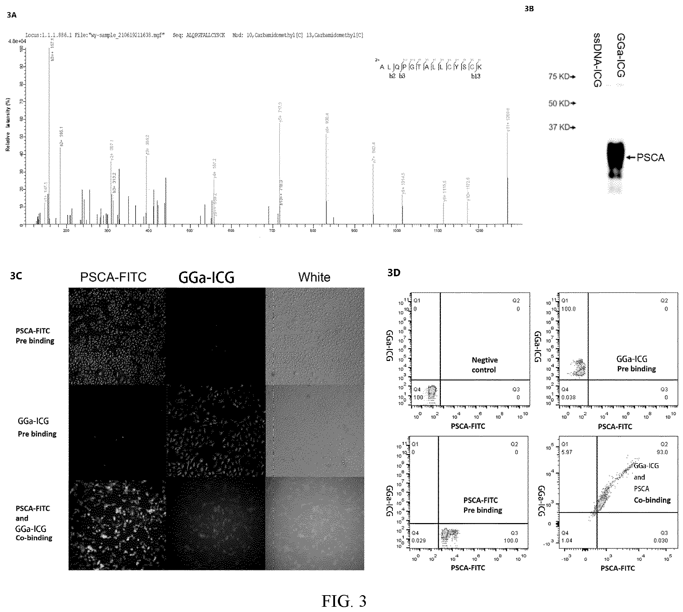

Figure 3 shows that PSCA is the target protein of GGa-ICG in the present invention. FIG. 3A indicates that PSCA is the target protein of GGa-ICG based on mass spectrometry analysis, FIG. 3B confirms the direct interaction between PSCA and GGa-ICG through pull-down assay, and FIGS.3C-3D demonstrate the competition between PSCA antibody and GGa-ICG through fluorescence microscopy and flow cytometry.

Figure 4 demonstrates the in vivo experiments of GGa-ICG. FIG.4A and 4C show the specific binding of GGa-ICG to prostate cancer xenografts in vivo, FIG.4B shows the in vivo distribution of GGa-ICG, and FIGS.4D-4G demonstrate the dose and time dependence in vivo imaging of GGa-ICG.

Figure 5 shows that PSCA completely blocks the imaging of GGa-ICG.

Figure 6 shows the staining of clinical specimens with GGa-ICG in the present invention.

Figure 7 demonstrates the specific imaging of GGa-ICG in multiple cancer types. FIG.7A shows the binding of GGa-ICG to different tumor cell lines through flow cytometry, and FIG.7B shows the in vivo specific binding of GGa-ICG to multiple cancer types.

## Detailed Description

The following description of the invention will be further illustrated in conjunction with the attached figures. It should be noted that the following examples are based on the technical scheme of the present invention, which provides detailed implementation methods and specific operating procedures. However, the scope of protection of the invention is not limited to these examples.

The present invention provides a method for synthesizing a near-infrared probe, GGa-ICG, which specifically binds to PSCA. The synthesis method includes the following steps :

S1:

Into a microwave vessel were added

1,1,2-trimethyl-1H-benzo[e]indole (1 mmol, 209 mg), 6-bromohexanoic acid (0.5 mmol,98 mg), potassium iodine (0.5 mmol, 83 mg) and a magnetic stir bar. The vessel was sealed with a septum and placed into the microwave cavity. The reaction mixture was kept at 145 °C for 90 min. The mixture was worked up and purified after reaction completion to afford the target compound 3-(5-carboxypentyl)-1,1,2-trimethyl-1H-benzo[e]indol-3-ium iodide as gray solids with 47% yield. $^1$H NMR (400 MHz, MeOD) δ 8.36 (d, $J$ = 8.5 Hz, 1H), 8.28 (d, $J$ = 9.0 Hz, 1H), 8.20 (d, $J$ = 8.2 Hz, 1H), 8.05 (d, $J$ = 8.9 Hz, 1H), 7.84 (ddd, $J$ = 8.4, 6.8, 1.3 Hz, 1H), 7.75 (ddd, $J$ = 8.1, 6.9, 1.1 Hz, 1H), 4.68 (t, $J$ = 7.7 Hz, 2H), 2.38 (t, $J$ = 7.1 Hz, 2H), 2.16 - 2.04 (m, 2H), 1.88 (s, 6H), 1.76 (p, $J$ = 7.2 Hz, 2H), 1.62 (tt, $J$ = 10.1, 6.0 Hz, 2H).

S2:

4-(1,1-dimethyl-1H-benzo[e]indol-3-ium-3-yl)butane-1-sulfonate (1 mmol, 345 mg) was dissolved in acetic anhydride (10 mL). Then N-((1E,3E,5E)-5-(phenylimino)penta-1,3-dien-1-yl)aniline (0.1 mmol, 28.5 mg) was added and the mixture was stirred at 120°C for 5 hours. After cooling to room temperature, 3-(5-carboxypentyl)-1,1,2-trimethyl-1H-benzo[e]indol-3-ium iodide (10 mmol, 4510 mg) was dissolved in pyridine (20 mL) was added dropwise. After dropping completion, the mixture was stirred at 50°C for 2 hours. The mixture was quenched by hydrochloric acid and subsequently purified to afford the target compound .4-(2-((1E,3E,5E,7E)-7-(3-(5-carboxypentyl)-1,1-dimethyl-1,3-dihydro-2H -benzo[e]indol-2-ylidene)hepta-1,3,5-trien-1-yl)-1,1-dimethyl-1H-benzo[e]indol-3-iu m-3-yl)butane-1-sulfonate as dark purple solids with 17% yield. 1H NMR (400 MHz, CDCl3) δ 8.07 (d, J = 8.6 Hz, 2H), 7.91 (dd, J = 13.8, 8.4 Hz, 4H), 7.82 (s, 1H), 7.72 (s, 1H), 7.58 (d, J = 9.7 Hz, 2H), 7.44 (dd, J = 16.3, 10.2 Hz, 4H), 7.34 (d, J = 8.8 Hz, 1H), 6.81 (s, 1H), 6.63 (d, J = 20.6 Hz, 2H), 6.13 (s, 1H), 4.28 (s, 2H), 4.17 - 3.98 (m, 2H), 3.14 (s, 8H), 2.49 (t, J = 7.2 Hz, 2H), 2.15 (d, J = 21.7 Hz, 4H), 1.80 (t, J = 7.3 Hz, 15H), 1.59 (d, J = 10.0 Hz, 3H).

S3:

4-(2-((1E,3E,5E,7E)-7-(3-(5-carboxypentyl)-1,1-dimethyl-1,3-dihydro-2H-benzo[e]in    dol-2-ylidene)hepta-1,3,5-trien-1-yl)-1,1-dimethyl-1H-benzo[e]indol-3-ium-3-yl)buta ne-1-sulfonate (1 mg), N-(3-dimethylaminopropyl)-N'-ethylcarbodiimide hydrochloride (EDCI, 10 mg) and 4-dimethylaminopyridine (DMAP, 10 mg) were dissolved in dichloromethane (DCM, 5 mL). GGa(NH2C6) (10 mg) or GGa(NH2C12) (12 mg) was suspended in DMF (1 mL) was added. After stirring at room temperature for 48 hours, the mixture was worked up and purified by dialysis and lyophilization to afford the target compound GGa-ICG as dark green solids with a grafting yield of approximately 10%.

The present invention also provides an application of the obtained near-infrared probe GGa-ICG in detecting PSCA, which can specifically bind to PSCA, be internalized by cells through the caveolin pathway, and can detect near-infrared fluorescence signals in target cells within 48 hours.

It should be noted that the penetration depth of the near-infrared fluorescence probe GGa-ICG can reach 6mm.

It should be noted that the binding site of the near-infrared probe GGa-ICG and PSCA protein is amino acids 14-28, i.e., ALQPGTALLCYSCKA.

It should be noted that the near-infrared fluorescence probe GGa-ICG can be used for the specific detection of total PSCA in prostate cancer, bladder cancer, kidney cancer, breast cancer, colon cancer, lung cancer, and pancreatic cancer.

**Embodiments**

After preparing the near-infrared fluorescent probe GGa-ICG using the method provided by this invention, the obtained GGa-ICG should be identified.

**Fluorescence titration**

The Horiba FluoroMax4 (Japan) fluorescence spectrometer and high-precision quartz ultra-micro cuvette (optical path: 10 mm × 4 mm) were used for fluorescence titration. The scan time for all spectra was 0.5 seconds, with emission and excitation slit widths of 1.3 nm and a step size of 1 nm. Fluorescence spectra were obtained between 630 nm and 900 nm and averaged over three scans or more. The emission wavelength of ICG and its derivatives was 810 nm. Fluorescence titration was performed by continuously adding concentrated GGa-ICG to the excitation wavelength, and the measurement value was obtained after 5 minutes of equilibration time. The fluorescence data were converted into a binding curve using the following equation to determine the excitation wavelength (where I is the intensity at the fluorescence maximum wavelength (750-830 nm) and $I^\lambda$ is the intensity at the free and fully bound ligand boundary):

$$\alpha = \frac{I - I_\lambda^{free}}{I_\lambda^{bound} - I_\lambda^{free}}$$

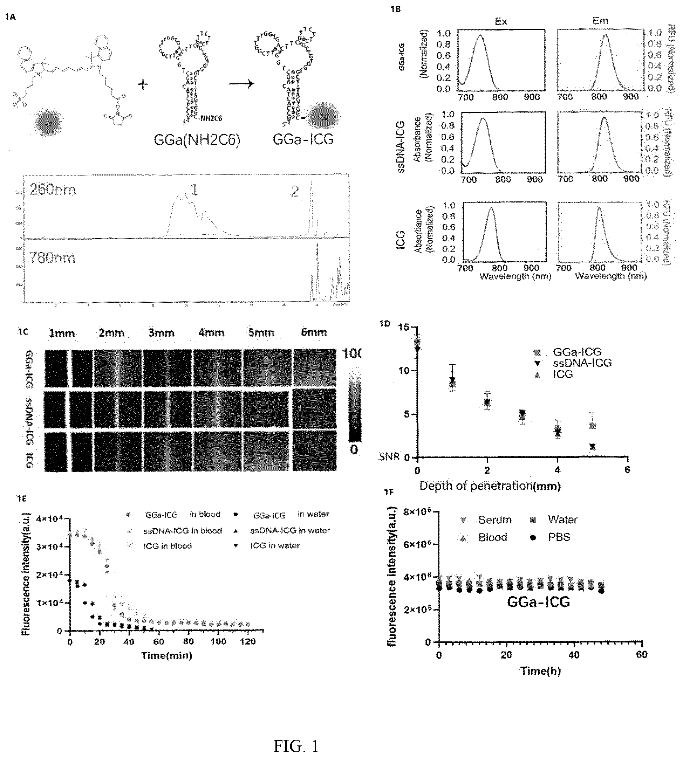

The results indicated that the excitation wavelength of GGa-ICG is 780nm, and the excitation wavelength of the control reagent ssDNA-ICG was the same as that of ICG, which was around 780nm (Figure 1C).

**Fluorescence depth detection**

The tissue penetration depth of ICG, GGa-ICG, and ssDNA-ICG was detected using a fluorescence spectrometer at depths of 1, 2, 3, 4, and 5 mm. Fluorescence was excited at a wavelength of 780 nm and fluorescence signals were detected after 5 minutes to compare signal-to-noise ratios and observe the fluorescence signal penetration depth.

The results, shown in Figures 1D-1E, indicate that the penetration depth of all three compounds was around 3 mm, while the penetration depth of GGa-ICG was close to 6 mm.

**Fluorescence stability detection**

Stability testing was divided into two groups: 1) stability testing under excitation wavelength and 2) stability testing of GGa-ICG under visible light. Excitation wavelength of 780 nm (GGa-ICG, ssDNA-ICG, and ICG) and visible light were used to excite the fluorescence probe GGa-ICG. Fluorescence detection was performed, and the changes in fluorescence intensity were recorded using a fluorescence spectrophotometer.

As shown in Figure 1F-1G, GGa-ICG, ssDNA-ICG, and ICG maintained maximum fluorescence intensity for 20-30 minutes after excitation at a wavelength of 780 nm. Fluorescence decay began at 30-40 minutes, and the fluorescence was extinguished after 1 hour. GGa-ICG did not exhibit fluorescence quenching under visible light excitation during the detection time.

Specific binding of GGa-ICG to prostate cancer cell lines

In previous studies, the nucleic acid aptamer sequence of GGa was confirmed to specifically bind to prostate cancer cells PC3, but not to other cells. Therefore, 200 nM of GGa-ICG and ssDNA-ICG were used to confirm their specific binding. The method is as follows:

a. The human prostate cancer cell lines PC-3, DU145, LNCaP, 22RV1, immortalized normal human prostate epithelial cell line RWPE1, and human cervical cancer cell line Hela, as well as MCF7 cells obtained from the American Type Culture Collection (ATCC), were used in this study. All cell lines were cultured at 37°C and 5% CO2 in a humidified environment. RWPE-1 cells were cultured in keratinocyte serum-free medium (K-SFM) supplemented with bovine pituitary extract (BPE) and human recombinant epidermal growth factor (EGF), while other tumor cell lines were cultured in RPMI 1640 medium supplemented with 10% fetal bovine serum (FBS, GIBCO) and 100 units/mL penicillin-streptomycin (Sigma).

b. DNA sequence and the buffer

GGa-ICG:

$$5'\text{-TGCCACTACAGCTGGTTCGGTTTGGTGACTTCGTTCTTCGTTGTG}$$

$$GTGCTTAGTGGC\text{-}3'\text{-ICG}$$

ssDNA-ICG:

$$5'\text{-ACGCTCGGATGCCACTACAG-45nt-CTCATGGACGTGCTGGTGAC-}$$

$$3'\text{-ICG}$$

The GGa and ssDNA libraries mentioned above were purchased from Sangon Biotech (Shanghai) Co., Ltd. (China).

Prepare a washing buffer by adding 4.5 g glucose and 5 mmol MgCl2 to 1 L of 0.01 M PBS pH 7.4 (8 g NaCl, 0.2 g KCl, 0.272 g KH2PO4, 3.58 g Na2HPO4·12H2O/1L ddH2O). The binding buffer is made by combining the washing buffer with 0.1 mg/mL salmon sperm DNA (Sigma) and 1 mg/mL bovine serum albumin (BSA, Sigma).

c. Cell binding

After the cells reach a confluency of over 80%, they were passaged and grown for 24-48 hours until they reached a confluency of over 50%. They were then washed once with PBS (without Ca and Mg ions), twice with cold washing buffer, and incubated at room temperature (4□ for the internalization inhibition group) for 30 minutes with the sample buffer containing near-infrared fluorescence probes GGa-ICG, ssDNA-ICG, and ICG to a final concentration of 200 nM (or as specified). The cells were observed under a fluorescence microscope or analyzed by flow cytometry after digestion with trypsin without EDTA.

d. Affinity detection

To test the affinity of different GGa-ICG aptamers for PC-3 cells, aptamers at different concentrations (0, 10, 20, 50, 100, 200, 500 nM) are incubated with $2 \times 10^5$ cells at 37°C. After digestion into a cell suspension, flow cytometry was used for analysis. The average fluorescence intensity of the background was subtracted from that of each sample. The equilibrium dissociation constant (Kd) for aptamer-cell interactions is calculated using SigmaPlot software.

e. MTT experiment

MTT assay was used to measure the in vitro cell toxicity in a 96-well plate. In brief, cells ($6 \times 10^3$ cells per well) were treated with GGa-ICG (at concentrations of 0, 20, 200 nM, 10 $\mu$M, etc.), free drugs, or hybrids in the culture medium (RPMI1640 without FBS, unless otherwise specified; 37°C; 5% CO2). After 4 hours, the cells are washed and fresh medium was added. The cells were then cultured for 48 hours, and cell viability was determined using the MTT assay according to the manufacturer's protocol.

f. Endocytosis experiment

PC-3 cells were incubated with 200 nM GGa-ICG or ssDNA-ICG in 200 $\mu$L binding buffer at 37°C for 10 minutes to observe cell membrane binding. After 2 hours of incubation, the cells were treated with 0.25% trypsin or 0.1 mg/mL proteinase K at 37°C for 10 minutes to observe internal binding.

For inhibition of endocytosis, PC-3 cells were incubated with 200 nM GGa-ICG or ssDNA-ICG in 200 $\mu$L binding buffer at 4°C for 2 hours to observe cell membrane binding. After 4 hours of incubation, the cells were treated with 0.25% trypsin or 0.1 mg/mL proteinase K at 37°C for 10 minutes to observe internal binding.

g. Mass spectrometry analysis of the target protein of GGa-ICG

PC-3 cells were washed three times with cold PBS buffer and lysed at 4°C for 30 minutes in 5 mL of low-salt buffer [50 mM Tris-HCl (pH=7.5) containing protease inhibitor (0.1 mM PMSF complex)]. After centrifugation, the pellets were washed 3 times with 5 mL of low-salt buffer and then solubilized in 1.5 mL of lysis buffer (PBS containing 5 mM MgCl2 and 1% Triton X-100) at 4°C for 30 minutes. The resulting supernatants were separately incubated with beads, ssDNA-ICG, or GGa-ICG at 4°C for 1 hour. Protein-ssDNA-ICG or protein-GGa-ICG complexes were then captured by incubating them with 2 mg (200 $\mu$L) of streptavidin beads at 4°C for 45 minutes. After centrifugation, the captured proteins were heated and eluted in 30 $\mu$L of loading buffer and then analyzed by SDS-PAGE gel and Coomassie blue staining. The protein bands showing significant differences in aptamer purification among different groups were subjected to in situ digestion and analyzed by LC-MS/MS.

h. Pull-down assay

Membrane proteins of PC-3 cells were extracted and incubated with agarose beads, agarose-conjugated GGa-ICG, or an agarose-conjugated library. The resulting pull-down mixture containing proteins was analyzed by Western blotting using a PSCA-specific antibody. The captured proteins were analyzed by SDS-PAGE gel and Coomassie blue staining, followed by Western blotting using an anti-PSCA antibody.

i. Flow cytometry

A suspension of $5 \times 10^5$ cells of PC-3 or other cell lines was incubated with 200 nM of GGa-ICG or anti-PSCA-FITC antibody in 100 $\mu$L of binding buffer on ice for 30 minutes, washed again, and subjected to flow cytometry analysis (FACS Calibur instrument, BD Biosciences); 10,000 events were collected for each sample. ICG-labeled ssDNA-ICG was used as a negative control. For the antibody competition experiment, $5 \times 10^5$ cells were incubated with 200 nM of GGa-ICG in binding buffer on ice for 30 minutes, followed by the addition of 10 $\mu$g/mL of anti-PSCA-FITC antibody and further incubation for 30 minutes as the GGa-ICG blocking group. 10 $\mu$g/mL of anti-PSCA-FITC was incubated in binding buffer on ice for 3 minutes, followed by the addition of 200 nM of GGa-ICG and further incubation for 30 minutes as the anti-PSCA-FITC blocking group. Both 200 nM of GGa-ICG and 10 $\mu$g/mL of anti-PSCA-FITC antibody were incubated on ice for 30 minutes as the combined group. After washing, the cells were resuspended and subjected to flow cytometry analysis.

**Results**

GGa-ICG specifically binds to prostate cancer cell lines

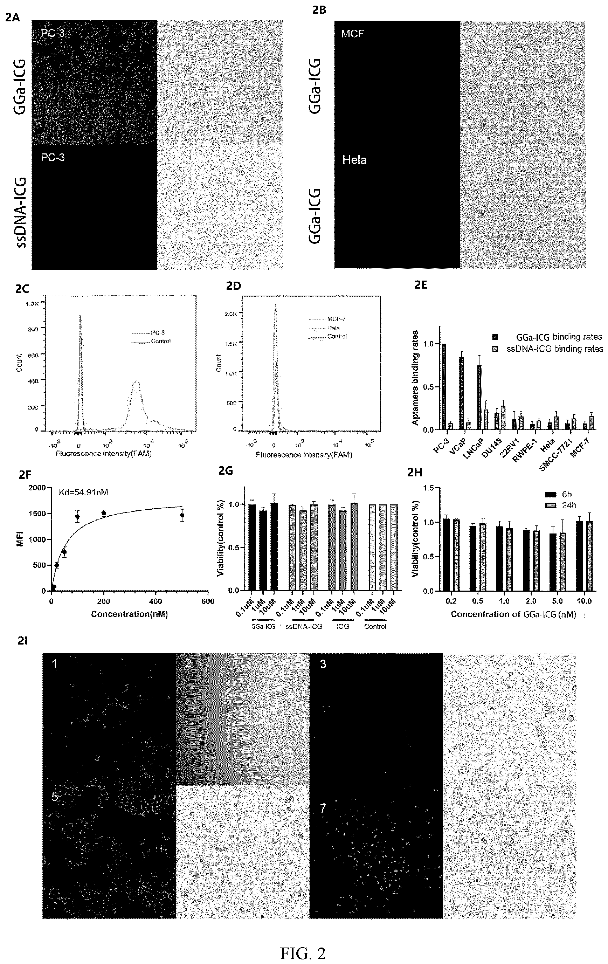

As shown in Figures.2A-2E, GGa-ICG could specifically bind to prostate cancer cell lines, and the binding affinity of PC3, VCap, and LNCap cell lines was stronger than that of DU-145, 22RV1 cells, RWPE-1, and MCF2, Hela cells had significantly reduced binding affinity.

As shown in Figure 2F, GGa-ICG had a high affinity for PC3 with a dissociation constant of 54.91nM.

GGa-ICG did not affect cell growth and was not toxic.

As shown in Figures 2G-2H, in the MTT experiment, a toxicology experiment was conducted for 24 hours with 2-20 times the Kd value concentration and 20 times the concentration, and no significant cell inhibition of GGa-ICG was observed.

As shown in Figure 2I, GGa-ICG enters cells through endocytosis.

In the internalization experiment, the bound probe on the cell membrane surface was washed off by trypsin, and after adjusting the fluorescence microscope plane, fluorescent probes for internalization were visible inside the cells. After incubation with the bound probe at 4°C, the fluorescent probe bound to the cell membrane could be completely washed off, and there was no fluorescent signal inside the cell, indicating that GGa-ICG enters cells through endocytosis rather than free diffusion.

PSCA as a target protein of GGa-ICG

Based on the above results, total membrane proteins were extracted from PC-3 cells and incubated with biotinylated GGa-ICG or ssDNA-ICG separately. Then, the binding complex was separated using streptavidin-coated magnetic beads and analyzed by SDS-PAGE gel. A characteristic protein band with a relative molecular weight of 35kDa was found in the GGa-ICG column, which was digested and analyzed by LC-MS/MS QSTAR. The MS results produced a protein hit list, with PSCA ranking first with the highest score and the most content among these candidates.

To confirm the results of the mass spectrometry, the location of GGa-ICG and PSCA on PC-3 cells was first examined. To achieve this, PC-3 cells were incubated with fluorescein isothiocyanate and anti-PSCA antibody conjugated with GGa-ICG. Flow cytometry showed a competitive relationship between GGa-ICG and PSCA antibody.

To determine whether GGa-ICG interacts with PSCA, a pull-down assay was used for further verification, confirming the direct interaction between GGa-ICG and PSCA. PSCA was easily pulled down by GGa-ICG but not by agarose beads or ssDNA, demonstrating the direct interaction between GGa-ICG and PSCA.

In vivo imaging of GGa-ICG

Establishment of mouse xenograft model

Male BALB/c nude mice (BALB/c-nude) were purchased from Beijing SLAC Laboratory Animal Co., Ltd. Five-week-old nude mice were subcutaneously injected with $5\times10^6$ PC-3 cells on the back. The tumor was allowed to grow for 15 to 20 days until it reached a diameter of 0.5-1.5 cm. The BALB/c nude mice bearing tumors were anesthetized with sedatives and anesthetics. Once the mice were immobilized, a designated dose (0.1 mg, 0.5 mg, 1 mg, 2 mg per injection) of GGa-ICG or ssDNA-ICG was injected through the tail vein. At certain time points, fluorescent images of live mice were collected using the IVIS Lumina II in vivo imaging system. For ex vivo fluorescent experiments, tumor-bearing mice that were intravenously injected with GGa-ICG or ssDNA-ICG were euthanized by cervical dislocation 1 hour after injection under anesthesia. After dissection, organs including brain, liver, kidney, spleen, lung, heart, and tumor tissue were imaged using the IVIS Lumina II in vivo imaging system as described above.

Results

GGa-ICG specifically recognizes tumor lesions in vivo

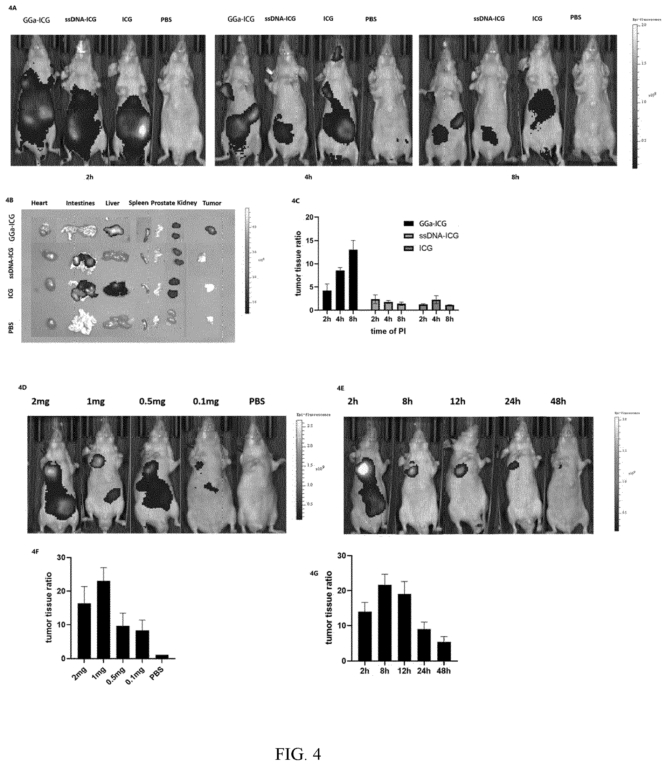

After intravenous injection into PC tumor-bearing mice, GGa-ICG, ssDNA-ICG, and common ICG specifically bound to prostate cancer lesions at 2, 4, and 8 hours, as shown in Figure 4A. Their organ distribution and time-dose curves are shown in Figures.4B-4G. GGa-ICG at doses of 0.1-2 mg/animal showed optimal detection at 8-12 hours after injection, and lasted until 24-48 hours. GGa-ICG in the organs was essentially metabolized within 24 hours.

In vivo blocking of GGa-ICG by PSCA antibodies

1 mg of PSCA antibody was pre-injected into nude mice, followed by injection of GGa-ICG 12 hours later, and live imaging was performed to observe the phenomenon of GGa-ICG binding.

Results

PSCA completely blocked the visualization of GGa-ICG in the tumor.

Toxicity test of GGa-ICG

Blood serum was collected from nude mice injected with GGa-ICG for 72 hours and subjected to enzymatic testing.

As shown in Table 1, GGa-ICG did not show in vivo toxicity.

## 72h Serological test

Preclinical application of GGa-ICG

Methods: Paraffin tissue blocks were provided by the Pathology Department of the Third Affiliated Hospital of Sun Yat-sen University (Guangzhou, China). Tissue sections were deparaffinized twice in xylene for 10 min each time, followed by rehydration in a series of graded ethanol (100%, 95%, and 70%) and rinsed in deionized water. After washing in PBS for 5 min, the sections were incubated in TE buffer (10 mM Tris, 1 mM EDTA, pH=8.0, per liter of ddH2O) and boiled in a microwave oven until boiling, then kept at 95□ for 15 min to retrieve the antigen. The sections were allowed to cool naturally to room temperature and then washed three times with fresh PBS. After histological processing, tissue sections were incubated with binding buffer containing 20% FBS and 0.1 mg/mL Herring Sperm DNA at room temperature for 60 min, followed by incubation with 200 nM GGa-ICG in the binding buffer at 4□ for 60 min. The sections were washed three times with fresh PBS, dehydrated, and sealed with Antifade PVA mounting medium. Finally, stained sections were imaged using a spinning disk confocal fluorescence microscope (Olympus IX71, Japan). Adjacent sections were stained with PSCA-FITC antibody for HE staining. Fluorescence signals were observed using a 40× objective lens (NA 0.90, UPLSAPO, Olympus, Japan). Images were analyzed using FV10-ASW 3.1 software.

Results: GGa-ICG imaging of PC clinical tissue.

Laser confocal fluorescence microscopy imaging analysis of GGa-ICG staining in PC cases and normal cases. Strong fluorescence signals were observed in PC and metastatic tissues after incubation with GGa-ICG. No fluorescence signal was detected in normal tissues, indicating the tumor-specificity of this fluorescent probe. The binding rate of GGa-ICG with PC tissues was 82.5% according to the comprehensive measurement results. Therefore, GGa-ICG has the potential to be an effective molecular diagnostic reagent for identifying PC tumor tissues.

Visualization of GGa-ICG in other types of cancer

PSCA is expressed at high levels in various tumors such as prostate cancer, bladder cancer, kidney cancer, breast cancer, colon cancer, lung cancer, pancreatic cancer, etc. Using the aforementioned method, T24 bladder cancer cells, Caki1 renal cancer cells, MDA-MB468 breast cancer cells, HT29 colon cancer cells, PANC1 pancreatic cancer cells, NCI-H460 lung cancer cells, and other cell lines were stained with GGa-ICG for flow cytometry analysis. Positive cell lines were used to establish subcutaneous transplantation tumor models to detect the visualization of GGa-ICG in vivo.

Results:

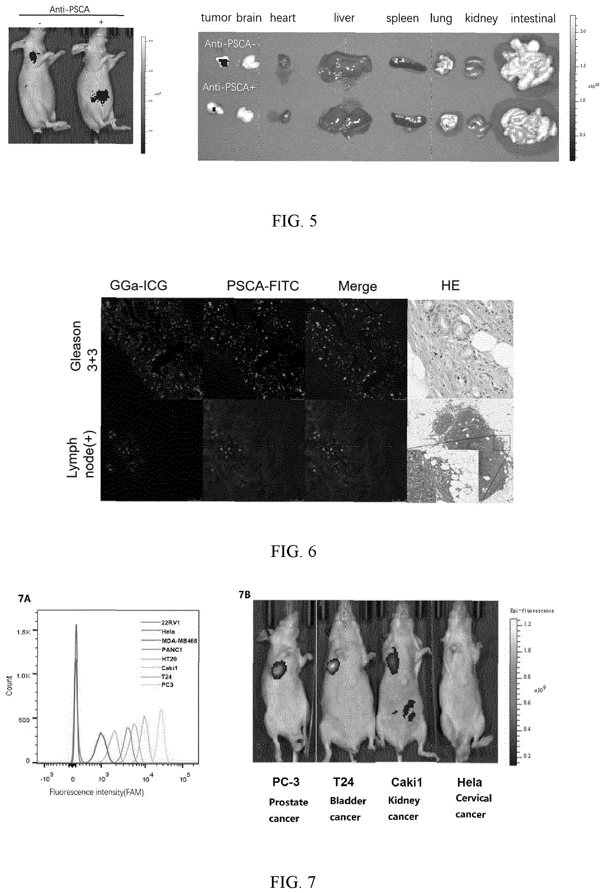

PSCA expression levels varied among different types of tumors. Prostate cancer, bladder cancer, kidney cancer, breast cancer, colon cancer, lung cancer, and pancreatic cancer showed high levels of PSCA expression, while cervical cancer and other tumors showed decreased expression levels. Flow cytometry results indicated that GGa-ICG could bind to tumor cell lines with elevated PSCA expression, as shown in Figure 7A; in vivo binding of GGa-ICG is consistent with PSCA expression, as shown in Figure 7B.

For those skilled in the art, various corresponding changes and deformations can be given according to the above technical scheme and conception, and all these changes and deformations should be included in the scope of protection of the claims of the invention.

## Claims

1.  A synthesis method of a PSCA-specific binding near-infrared probe GGa-ICG, wherein the synthesis method includes the following steps:

    S1: Into a microwave vessel were added 1,1,2-trimethyl-1H-benzo[e]indole, 7-bromohexanoic acid, potassium iodine and o-dichlorobenzene. The vessel was sealed with a septum and placed into the microwave cavity. The reaction mixture was kept at 145 °C for 90 min. The mixture was worked up and purified after reaction completion to afford the target compound 3-(5-carboxypentyl)-1,1,2-trimethyl-1H-benzo[e]indol-3-ium iodide as gray solids with 47% yield.
    S2: 4-(1,1-dimethyl-1H-benzo[e]indol-3-ium-3-yl)butane-1-sulfonate was dissolved in acetic anhydride. Then N-((1E,3E,5E)-5-(phenylimino)penta-1,3-dien-1-yl)aniline was added and the mixture was stirred at 120°C for 5 hours. After cooling to room temperature, 3-(5-carboxypentyl)-1,1,2-trimethyl-1H-benzo[e]indol-3-ium iodide was dissolved in pyridine was added dropwise. After dropping completion, the mixture was stirred at 50°C for 2 hours. The mixture was quenched by hydrochloric acid and subsequently purified to afford the target compound 4-(2-((1E,3E,5E,7E)-7-(3-(5-carboxypentyl)-1,1-dimethyl-1,3-dihydro-2H-benzo[e]in     dol-2-ylidene)hepta-1,3,5-trien-1-yl)-1,1-dimethyl-1H-benzo[e]indol-3-ium-3-yl)buta ne-1-sulfonate as purple red solid with 17% yield.
    S3:4-(2-((1E,3E,5E,7E)-7-(3-(5-carboxypentyl)-1,1-dimethyl-1,3-dihydro-2H-benzo[e ]indol-2-ylidene)hepta-1,3,5-trien-1-yl)-1,1-dimethyl-1H-benzo[e]indol-3-ium-3-yl)b utane-1-sulfonate, N-(3-dimethylaminopropyl)-N'-ethylcarbodiimide hydrochloride (EDCI) and N-Hydroxysuccinimide(NHS) were dissolved in dichloromethane (DCM). After stirring at room temperature for 5 hours, the reaction was worked up and purified to afford afford the target compound
    2-[(1E,3E,5E)-7-[(2E)-3-{6-[(2,5-dioxypyrrolidine-1-yl)O]-6-(caproyl)}-1,1-dimethyl  -1H,2H,3H-benzo[E]indole-2-ylidene]penta-1,3,5-triene-1-yl]-1,1-dimethyl-3-(4-sulfo natobutyl)-1H-benzo[E]indole-3-azole as dark green solid with 42% yield.
    S4:2-[(1E,3E,5E)-7-[(2E)-3-{6-[(2,5-dioxypyrrolidine-1-yl)O]-6-(caproyl)}-1,1-dime   thyl-1H,2H,3H-benzo[E]indole-2-ylidene]penta-1,3,5-triene-1-yl]-1,1-dimethyl-3-(4-s              ulfonatobutyl)-1H-benzo[E]indole-3-azole, GGa($NH_2C_6$) were dissolved in the buffer solution ((pH= 9.18).The suspension was stirring at room temperature for 24 hours. After reaction, the mixture was purified by dialysis and lyophilization to afford the target compound GGa-ICG as a dark green solid.

2.  An application of the near infrared probe GGa-ICG obtained according to claim 1 to PSCA detection, wherein the near infrared probe GGa-ICG can be specifically bind to the PSCA, be endocytosed by cells via caveolin, and the near infrared fluorescence signals can be detected within 48 hours in target cells.

3.  The application of the near-infrared probe GGa-ICG to PSCA detection according to claim 2, wherein the penetration depth of the described near infrared fluorescence probe GGa-ICG can reach 6 mm.

4.  The application of the near-infrared probe GGa-ICG to PSCA detection according to claim 2, wherein the binding site of the described near-infrared probe GGa-ICG to PSCA protein is located in 14-28 amino acids, i.e., ALQPGTALLCYSCKA.

5.  The application of the near-infrared probe GGa-ICG to PSCA detection according to claim 2, wherein the described near-infrared fluorescence probe GGa-ICG can be used for the specific detection of total PSCA in prostate cancer, bladder cancer, kidney cancer, breast cancer, colon cancer, lung cancer and pancreatic cancer.

FIG. 1

FIG. 2

FIG. 3

FIG. 4

FIG. 5

FIG. 6

FIG. 7